# EUROPEAN PATENT SPECIFICATION

(11) **EP 1 620 552 B1**
(45) Date of publication and mention of the grant of the patent: **19.09.2012**
(21) Application number: 04759876.8
(22) Date of filing: 14.04.2004
(51) Int. Cl.: C12N 9/48

(54) **USE OF GLYCODENDRIMER REAGENTS FOR INHIBITING ADHESION BY MICROORGANISMS**
VERWENDUNG VON GLYCODENDRIMER-REAGENTIEN ZUR HEMMUNG DER ADHÄSION VON MIKROORGANISMEN
UTILISATION DE REACTIFS GLYCODENDRIMERES POUR L'INHIBITION DE L'ADHERENCE PAR DES MICRO-ORGANISMES

(30) Priority: 16.04.2003 US 417768
(43) Date of publication of application: 01.02.2006
(73) Proprietor: Danisco US Inc., Palo Alto, CA 94304 (US); THE GOVERNING COUNCIL OF THE UNIVERSITY OF TORONTO, Toronto, Ontario M5S 1A1 (CA); THE CHANCELLOR, MASTERS AND SCHOLARS OF THE UNIVERSITY OF OXFORD, Oxford OX1 2JD (GB)
(72) Inventor: BOTT, Richard, R., Burlingame, CA 94010 (US); COWAN, M., M., Cincinnati, OH 45246 (US); DAVIS, Benjamin, G., Durham, DH1 1RT (GB); JONES, John, Bryan, Lakefield, Ontario KO1 2HO (CA)
(74) Representative: Kremer, Simon Mark
(86) International application number: PCT/US2004/011605
(87) International publication number: WO 2004/093787

(56) References cited:
- WO-A-02/079394
- US-A- 6 159 447
- US-A1- 2002 019 039
- RENDLE PHILLIP M ET AL: "Glycodendriproteins: a synthetic glycoprotein mimic enzyme with branched sugar-display potently inhibits bacterial aggregation." JOURNAL OF THE AMERICAN CHEMICAL SOCIETY. 21 APR 2004, vol. 126, no. 15, 21 April 2004 (2004-04-21), pages 4750-4751, XP002379390 ISSN: 0002-7863
- LUNDQUIST JOSEPH J ET AL: "The cluster glycoside effect." CHEMICAL REVIEWS. FEB 2002, vol. 102, no. 2, February 2002 (2002-02), pages 555-578, XP002379392 ISSN: 0009-2665
- DAVIS BENJAMIN G ET AL: "Selective protein degradation by ligand-targeted enzymes: towards the creation of catalytic antagonists." CHEMBIOCHEM : A EUROPEAN JOURNAL OF CHEMICAL BIOLOGY. 6 JUN 2003, vol. 4, no. 6, 6 June 2003 (2003-06-06), pages 533-537, XP002379393 ISSN: 1439-4227
- DAVIS B G ET AL: "Altering the specificity of subtilisin Bacillus lentus through the introduction of positive charge at single amino acid sites." BIOORGANIC & MEDICINAL CHEMISTRY. NOV 1999, vol. 7, no. 11, November 1999 (1999-11), pages 2303-2311, XP000892841 ISSN: 0968-0896
- DAVIS B G ET AL: "The controlled introduction of multiple negative charge at single amino acid sites in subtilisin Bacillus lentus." BIOORGANIC & MEDICINAL CHEMISTRY. NOV 1999, vol. 7, no. 11, November 1999 (1999-11), pages 2293-2301, XP000892840 ISSN: 0968-0896

## Description

### Related Application Data

This application is a continuation-in-part of United States Application Serial Number 09/824,827 filed April 2, 2001, which claims priority to United States Application Serial Number 09/347,029 filed on July 2, 1999, United States Application Serial Number 60/131,446 filed on April 28,1999, United States Application Serial Number 60/091,687 filed on July 2, 1998.

### Field of the Invention

The present invention is directed to glyodendrimeric proteases, compositions with glycodendrimeric proteases and methods for inhibiting adhesin binding in microorganisms by contacting microorganisms with a glycodendrimeric protease or a composition with a glycodendrimeric protease. The invention may be used to treat patients in need of treatment.

### Background of the Invention

The emergence of drug-resistance among some pathogenic microorganisms necessitates a search for alternative methods to battle infections. Investigators have discovered and studied agents toxic to infecting microorganisms. Some investigators seek to fight disease by interrupting early stages of infection. Early stages include adhesion by a microorganism to a host tissue, which can be a prerequisite to establishing a harmful infection.

Adhesion can be mediated by microbial surface glycoproteins that act as markers in cell-cell communication events that determine microbial virulence (Sharon et al., Essays Biochem., 30:59-75 (1995)), inflammation (Lasky, Annu. Rev. Biochem., 64:113-139 (1995); Weis et al., Annu. Rev. Biochem., 65:441-473 (1996)), and host immune responses (Varki, Glycobiol., 3:97-130 (1993); Dwek, Chem. Rev., 96:683-720 (1996)). In addition, the correct glycosylation of proteins is critical to their expression and folding (Helenius, Mol. Biol. Cell, 5:253-265 (1994)) and increases their thermal and proteolytic stability (Opendakker et al., FASEB J., 7:1330-1337 (1993)). Glycoproteins occur naturally in a number of forms (glycoforms) (Rademacher et al., Annu. Rev. Biochem., 57:785-838 (1988)) that possess the same peptide backbone, but differ in both the nature and site of glycosylation.

Known strategies for reducing adhesion by a microorganism include using carbohydrate analogs to inhibit interactions between an adhesin molecule on a microorganism and a sugar on a host cell or tissue. (Zopf, D. et al., Adv. Exp. Med. Biol. 408:35-8 (1996); Ofek, I. and Doyle R.J., Bacterial Adhesion to Cells and Tissues; Chapman and Hall, New York (1994)). These analogs can be part of a carbohydrate cocktail, which includes carbohydrates having various structures corresponding to the binding specificities of one or more lectins of a microorganism. (Beuth, J. et al., Adv. Exp. Med. Biol. 408:51-56 (1996)). Manipulating gene regulation to prevent phenotype switching by the microorganism to an adhesion-plus variant provides yet another strategy for reducing adhesion by a microorganism. (Kahane, I. et al., Adv. Exp. Med. Biol. 408:107-111 (1996)).

For example, investigators have reported that, in the oral cavity, *Streptococcus mutans* attaches to glucans deposited on the tooth surface. (Koga, T. et al., J. Gen. Microbiol. 132:2873-2883 (1986)). Such attachment is believed to enhance the ability of *S*. *mutans* to metabolize dietary sucrose to acid, which then can destroy tooth enamel and eventually result in a carious lesion. *S*. *mutans* and other oral streptococci use a surface protein called glucan-binding lectin (GBL) to attach to surface-bound glucan. (Gibbons, R. J. et al., J. Bacteriol. 98:341-346 (1969)). Drake *et al.* developed an *in vitro* model system using soluble high-molecular weight glucans, also know as "dextrans," and whole cell suspensions of S. *cricetus* to examine GBL binding. (Drake, D. et al., Infect. Immun. 56:1864-1872 (1988); Drake, D. et al., Infect. Immun. 56:2205-2207 (1988)). The glucan binding results in aggregation that is quantifiable with spectrophotometry.

WO 02/079394 discloses the synthesis of chemically modified mutant protein, called also glycodendrimeric proteases; the subject matter of the application is also disclosed in US 02/10903.

Given the prevalence of harm caused by infection with microorganisms, and the medical effort and cost devoted to fighting these infections, there is a need for additional and improved compositions and methods for fighting infection by microorganisms. There is also a need for additional and improved compositions and methods for reducing adhesion by microorganisms to and in animal tissues, and for reducing adhesion by microorganisms to dental prostheses.

### Summary of the Invention

The present invention is directed to glyodendrimeric proteases, composition with glycodendrimeric proteases and methods for inhibiting adhesion of microorganisms by contacting microorganisms with a glycodendrimeric protease or a composition with a glycodendrimeric protease.

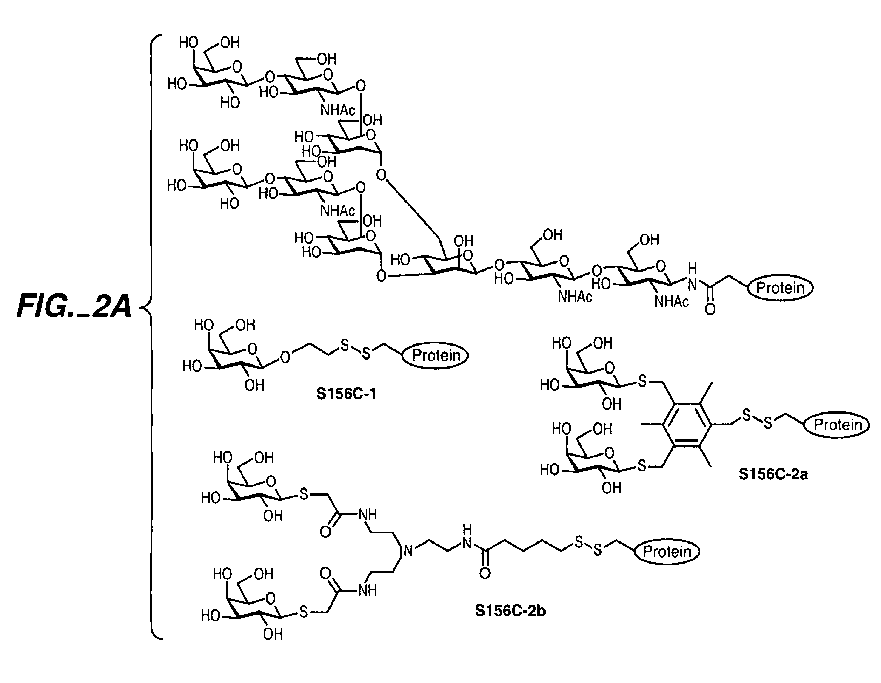

A first embodiment of the invention comprises a glyodendrimeric protease, the glycodendrimeric protease having a branched or multiply-branched structure that presents a carbohydrate moeity at each branch extremity wherein the glycodendrimeric protease inhibits adhesion of one or more microorganisms to a surface wherein the glycodendrimer protease has the structure shown in Fig. 2A as S156C-2b and wherein the protease is subtilisin Bacillus lentus S156C. Other carbohydrates are described in WO 02/079394. The surface may be selected from the group consisting of other bacterial cells, human or animal cells, tissues, extracellular matrix, teeth and prostheses. The microorganism may be a prokaryote, a eukaryote, a virus or a combination thereof. The prokaryote may be a gram-positive bacterium, a gram-negative bacterium or a gram-variable bacterium. The prokaryote may be an *Actinomyces, Staphylococcus* or an E. *coli.* The eukaryote may be a fungus or protozoan. The fungus may be *Candida.* The glycodendrimeric protease may inhibit adhesion by binding to a microbial adhesin, preferably a lectin, and causing the degradation of the proteinaceous lectin, among other mechanisms.

A second embodiment of the invention is drawn to a composition comprising, the glycodendrimeric protease of the first embodiment.

Described herein is a method for inhibiting adhesin binding in a microorganism comprising contacting the adhesin with a glycodendrimeric protease having a branched or multiply-branched structure that presents a carbohydrate moeity at each branch extremity wherein the glycodendrimeric protease inhibits adhesion of one or more microorganisms to a surface. The carbohydrate moeity is at least one selected from the group consisting of glucose, galactose, fucose, mannose, sialic acid, N-Acetylgalactosamine, N-Acetylglucosamine, and glucuronic acid. Other preferred carbohydrates can be found, for example, in WO 02/079394. The surface may be selected from the group consisting of other bacterial cells, human or animal cells, tissues, extracellular matrix, teeth and prostheses. The microorganism may be a prokaryote, a eukaryote, a virus or a combination thereof. The prokaryote may be a gram-positive bacterium, a gram-negative bacterium or a gram-variable bacterium. The prokaryote may be an *Actinomyces, Staphylococcus* or an *E*. *coli.* The eukaryote may be a fungus or protozoan. The fungus may be *Candida.* The glycodendrimeric protease may inhibit adhesion by binding to a microbial adhesin, preferably a lectin, and causing the degradation of the proteinaceous lectin, among other mechanisms.

Also described herein is a method of reducing binding of a microorganism to a surface comprising contacting the surface with a glycodendrimeric protease having a branched or multiply-branched structure that presents a carbohydrate moeity at each branch extremity wherein the glycodendrimeric protease inhibits adhesion of one or more microorganisms to a surface. The carbohydrate moeity is at least one selected from the group consisting of glucose, galactose, fucose, mannose, sialic acid, N-Acetylgalactosamine, N-Acetylglucosamine, and glucuronic acid. Other preferred carbohydrates can be found, for example, in WO 02/079394. The surface may be selected from the group consisting of other bacterial cells, human or animal cells, tissues, extracellular matrix, teeth and prostheses. The microorganism may be a prokaryote, a eukaryote, a virus or a combination thereof. The prokaryote may be a gram-positive bacterium, a gram-negative bacterium or a gram-variable bacterium. The prokaryote may be an *Actinomyces, Staphylococcus* or an *E*. *coli.* The eukaryote may be a fungus or protozoan. The fungus may be *Candida.* The glycodendrimeric protease may inhibit adhesion by binding to a microbial adhesin, preferably a lectin, and causing the degradation of the proteinaceous lectin, among other mechanisms.

Further described herein is a method of reducing adhesion by a microorganism to mammalian tissues or cells comprising contacting the mammalian tissues or cells with a glycodendrimeric protease having a branched or multiply-branched structure that presents a carbohydrate moeity at each branch extremity wherein the glycodendrimeric protease inhibits adhesion of one or more microorganisms to a surface. The carbohydrate moeity is at least one selected from the group consisting of glucose, galactose, fucose, mannose, sialic acid, N-Acetylgalactosamine, N-Acetylglucosamine, and glucuronic acid. Other preferred carbohydrates can be found, for example, in WO 02/079394. The surface may be selected from the group consisting of other bacterial cells, human or animal cells, tissues, extracellular matrix, teeth and prostheses. The microorganism may be a prokaryote, a eukaryote, a virus or a combination thereof. The prokaryote may be a gram-positive bacterium, a gram-negative bacterium or a gram-variable bacterium. The prokaryote may be an *Actinomyces, Staphylococcus* or an *E*. *coli.* The eukaryote may be a fungus or protozoan. The fungus may be *Candida.* The glycodendrimeric protease may inhibit adhesion by binding to a microbial adhesin, preferably a lectin, and causing the degradation of the proteinaceous lectin, among other mechanisms.

Described herein is a method of treating a disease or disorder of a patient in need of such treatment comprising administering to the patient a glycodendrimeric protease having a branched or multiply-branched structure that presents a carbohydrate moeity at each branch extremity wherein the glycodendrimeric protease inhibits adhesion of one or more microorganisms to a surface. The carbohydrate moeity is at least one selected from the group consisting of The carbohydrate moeity is at least one selected from the group consisting of glucose, galactose, fucose, mannose, sialic acid, N-Acetylgalactosamine, N-Acetylglucosamine, and glucuronic acid. Other preferred carbohydrates can be found, for example, in WO 02/079394. The surface may be selected from the group consisting of other bacterial cells, human or animal cells, tissues, extracellular matrix, teeth and prostheses. The microorganism may be a prokaryote, a eukaryote, a virus or a combination thereof. The prokaryote may be a gram-positive bacterium, a gram-negative bacterium or a gram-variable bacterium. The prokaryote may be an *Actinomyces, Staphylococcus* or an *E. coli.* The eukaryote may be a fungus or protozoan. The fungus may be *Candida.* The glycodendrimeric protease may inhibit adhesion by binding to a microbial adhesin, preferably a lectin, and causing the degradation of the proteinaceous lectin, among other mechanisms. The patient may be an animal or a human. Administration can be accomplished by any method suitable for delivering the glycodendrimeric protease to the site of the disease or disorder. For example, administration of the enzyme to the animal or human can be oral or topical. Administration can optionally be targeted to mammalian tissues infected by tissue destroying pathogens, or to the nose, ear, vagina, skin, lungs or digestive tract of the mammal.

Also described herein is a method for reducing adhesion by a microorganism to mammalian oral tissues or cells or to a dental prosthesis comprising providing glycodendrimeric protease having a branched or multiply-branched structure that presents a carbohydrate moeity at each branch extremity wherein the glycodendrimeric protease inhibits adhesion of one or more microorganisms to a surface. The carbohydrate moeity is at least one selected from the group consisting of glucose, galactose, fucose, mannose, sialic acid, N-Acetylgalactosamine, N-Acetylglucosamine, and glucuronic acid. Other preferred carbohydrates can be found, for example, in WO 02/079394. The surface may be selected from the group consisting of other bacterial cells, human or animal cells, tissues, extracellular matrix, teeth and prostheses. The microorganism may be a prokaryote, a eukaryote, a virus or a combination thereof. The prokaryote may be a gram-positive bacterium, a gram-negative bacterium or a gram-variable bacterium. The prokaryote may be an *Actinomyces, Staphylococcus* or an *E*. *coli.* The eukaryote may be a fungus or protozoan. The fungus may be *Candida.* The glycodendrimeric protease may inhibit adhesion by binding to a microbial adhesin, preferably a lectin, and causing the degradation of the proteinaceous lectin, among other mechanisms. The patient may be an animal or a human. Administration can be accomplished by any method suitable for delivering the glycodendrimeric protease to the site of the disease or disorder. For example, administration of the enzyme to the animal or human can be oral or topical. Administration can optionally be targeted to mammalian tissues infected by tissue destroying pathogens, or to the nose, ear, vagina, skin, lungs or digestive tract of the mammal. The method may include administering to a mammal's oral cavity an oral care composition including an effective amount of the glycodendrimeric protease. Advantages of this method can include reducing adhesion by one or more microorganisms to teeth; reducing dental caries, plaque or calculus; reducing co-aggregation of microorganisms; reducing pellicle formation, inhibiting glucosyltransferase or a combination thereof.

An embodiment of the invention provides an oral care composition comprising an effective amount of glycodendrimeric protease according to the first embodiment. Oral care compositions of the invention include, but are not limited to, a mouthwash, a toothpaste, an implant or a combination thereof, and may optionally be in the form of a solid, a semi-solid, a liquid or an aerosol. Administration of the oral care composition to the mammal's oral cavity can be accomplished by any method suitable for delivering a composition to the oral cavity. For example, administration of the oral care composition can include rinsing with a liquid, applying a semisolid with a toothbrush, swab or syringe, implanting a solid or a combination thereof. Optionally, the oral care composition can be used to treat a dental prosthesis, either in the oral cavity or outside the oral cavity. Such a treatment can include applying to a dental prosthesis removed from a mammal's oral cavity an oral care composition including an effective amount of the composition.

### Brief Description of the Drawings

Figure 1 shows a shows a proposed mechanism of action for glycodendrimeric proteases of the present invention;

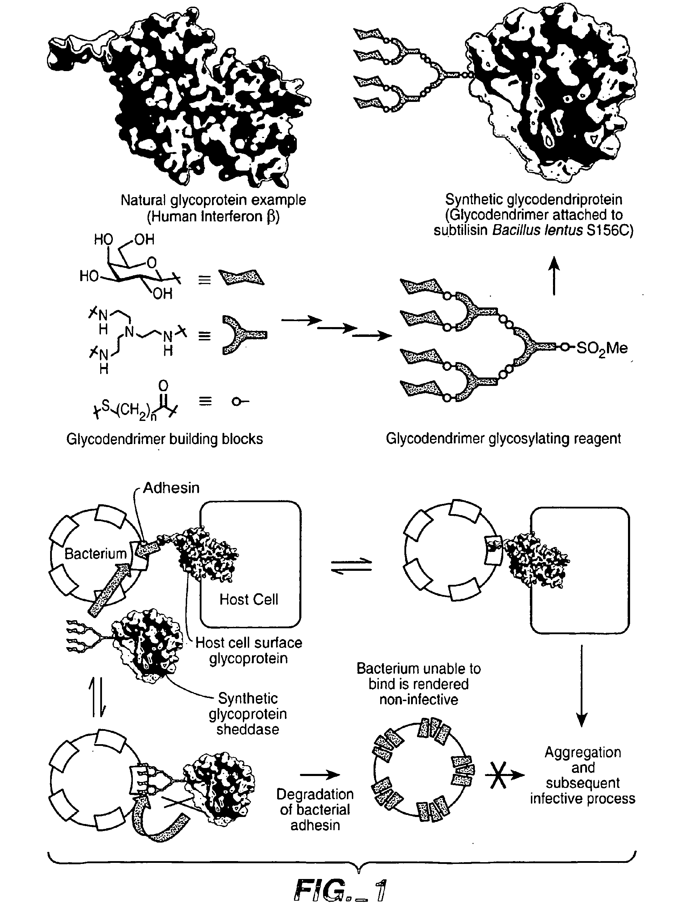

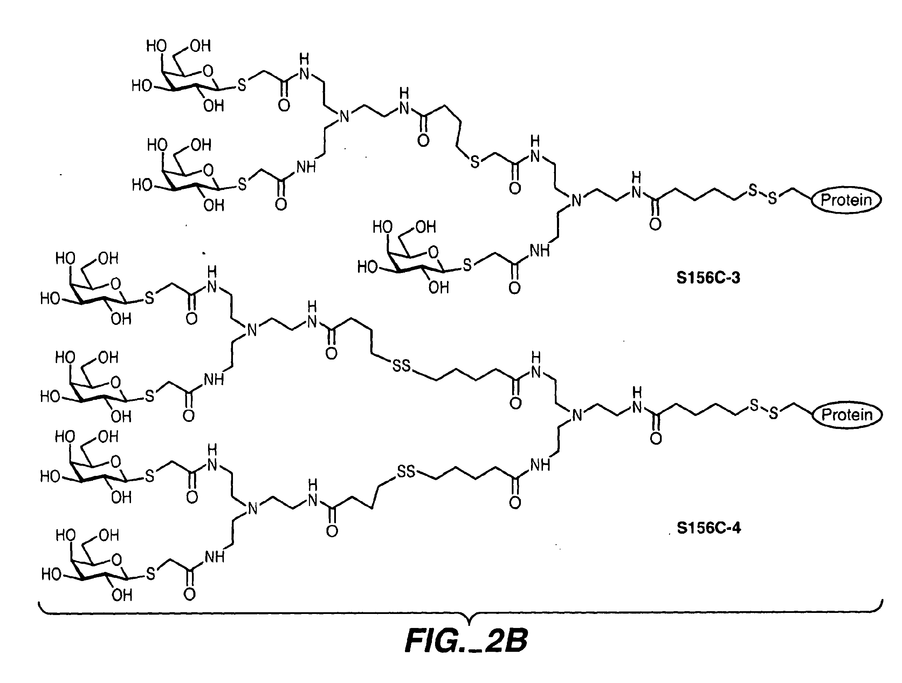

Figure 2 shows a proposed synthesis for glycodendrimeric proteases. **1-4.** MTS carbohydrate reagents **1-4** were used to modify the introduced cysteine thiol group (Figure 1a). One mono-antennary carbohydrate structure **1**(*21*) and four multi-antennary structures **2-4** were constructed (*35*) To explore the effect of branching substructure the core branching structures **of 2-4** were based upon both the conformationally flexible tris(2-aminoethyl)amine (TREN) core **6** to create **2b, 3, 4** and the rigid mesitylene core **5** to create **2a** (Figure 2). Since our model target pathogen, *A. naeslundii,* utilizes galactose (Gal)-specific binding adhesins(*36*) to bind Gal-containing glycoproteins,(*37*) Gal units were attached to the extremities of these dendrimer cores using 1-thio-®-Dgalactopyranose (Gal-SH) as a reagent. Through appropriate sequential use of the branched building blocks **7, 9,** and **11** and Gal-SH, the basic multiantennary structures **8, 10** and **12** were constructed. **8, 10** and **12** were then elaborated using the novel heterobifunctional linker reagent NHS-butyl-MTS to create a final amide bond using *N*hydroxysuccinimide (NHS) activated esters to give glycodendrimer reagents **2b, 3** and **4.** While reagents **2a, 2b** and **4** are symmetrical, this methodology also allowed the synthesis of an asymmetric tri-antennary reagent **3**, designed to mimic the asymmetric carbohydrate display that is observed in tri-antennary *N*-linked glycoproteins.(*38*) It is noteworthy that this chemistry was conducted completely without the use of carbohydrate protecting groups;

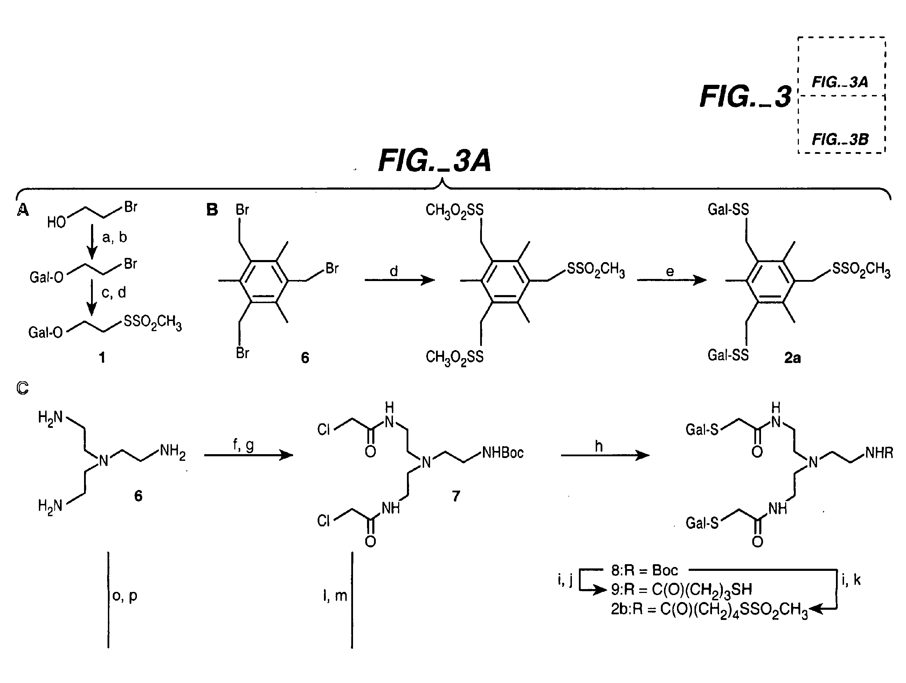

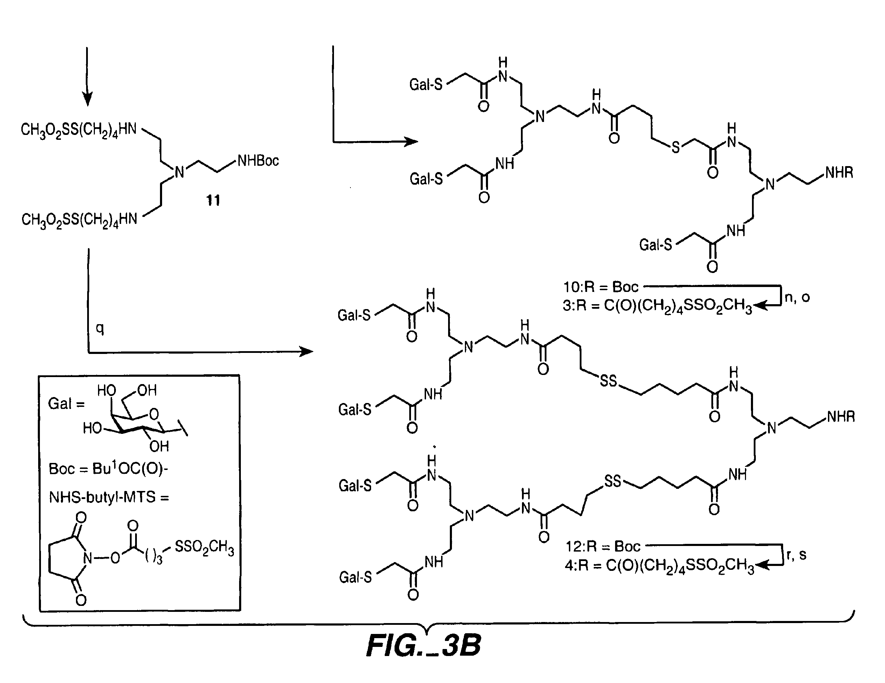

Figure 3 shows additional synthetic steps in synthesizing a glycodendrimeric protease.
   . **A**, **a**, pentaacetyl-Gal, BF3.Et2O, DCM, 76%; **b**, MeONa, MeOH, 83%; c, NaSSO2CH3, DMF, 78%; see Ref. (*21*) **B, d,** NaSSO2CH3, DMF, 56%; **e**, 2 equiv. Gal-S-Na+, DMF, 0°C 55%. **C, f,** Boc2O, DCM, -78°C, 68%; **g**, (ClCH2CO)2O, DCM, 97%; **h,** 2 equiv. Gal-S-Na+, DMF, 88%; **i,** CF3COOH, DCM, 91%; **j,** thiobutyrolactone, dithiothreitol, NaHCO3, water, EtOH, 69% **k,** NHS-butyl-MTS, DMF, 81%; **l**, 1 equiv. Gal-S-Na+, DMF, 48%; **m,** 11 **9**, DMF, 78%; **n,** CF3COOH, DCM, 94%; **o**, NHS-butyl-MTS, DMF, 77%; **p,** NHS-butyl MTS, DMF, 87%; **q, 9,** DMF, 81%; **r,** CF3COOH, DCM, 93%; **s**, NHS-butyl-MTS, DMF, 72%;

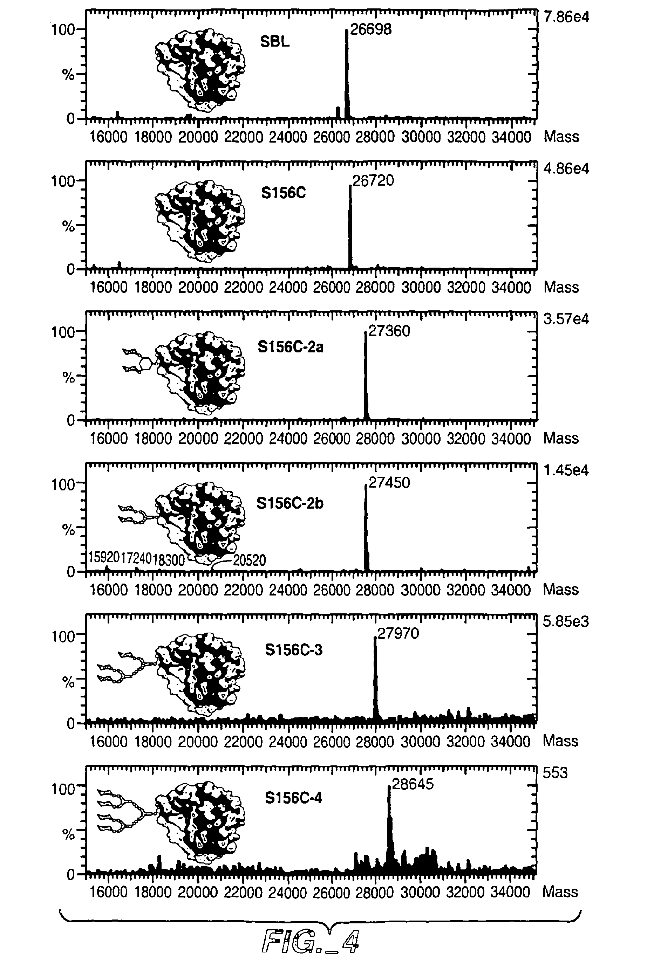

Figure **4** shows mass spectrometric characterization of glycoproteins. ESMS analysis confirmed the formation, with correct mass, of the expected glycoproteins S156C-**2a**, **2b**, **3**, **4**;

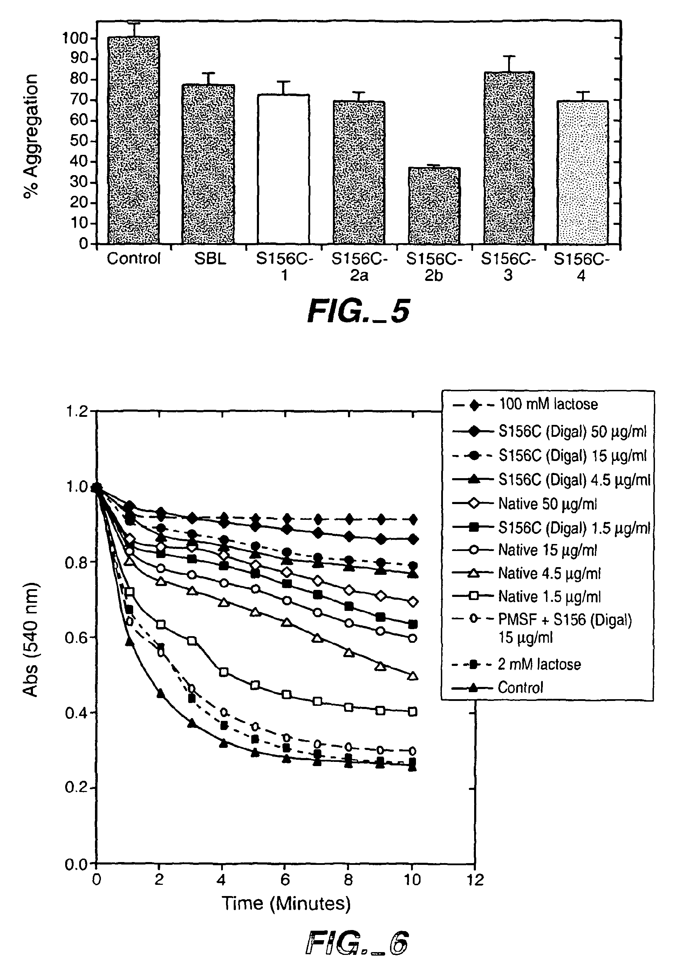

Figure 5 shows percent aggregation of the pathogen *A. naeslundii* to *S. oralis* (after 10 min) after treatment with glycoprotein enzyme (1.5µg/mL, 30 min). Most potent inhibition of the binding of *A. naeslundii* was found following treatment with the biantennary synthetic glycoprotein S156C-**2b** (IC50 20nM) and is >10⁶ more potent than that of small molecule inhibitor lactose (IC50 33mM);
Figure 6 shows results from a coaggregation assay used to demonstrate the principle of the invention. *Actinomyces naeslunii* T14V and *Streptococcus oralis* 34 were grown in Todd Hewitt broth supplemented with 2% yeast extract and grown at 37 ° C in 5% CO₂ and H₂:CO₂:N₂ (10:10:80) respectively, and harvested in mid- to late- exponential phase by centrifugation at 10,000 x g at 4° C for 10 min. Bacteria were washed twice and resuspended to A₅₄₀ = 1.0 in coaggregation buffer (CB: 1 mM tris (hydroxymethl) aminomethane, 10⁻⁴ M CaCl₂ , 10⁻⁴ M MgCl₂, 0.15 M NaCl, pH 7.0).
   Cell suspensions of *A. naeslundii* T14V were incubated with lactose, modified-proteases (S156C (1-4)), native protease, PMSF + S156C (1-4) and PMSF alone at 37°C for 1hr. After incubation an equal volume of *S. oralis* 34 was added and vortexed for 10 sec. Coaggregation was assessed by turbidity changes at 540 nm over 10 min - decreased turbidity indicating increased aggregation.
   This figure displays the concentration dependence of inhibition by the S 156C biantennary glycodendrimer, compared to native protease without galactose substitution. 100 mM lactose (digalactoside) results in no loss of turbidity (i.e. results in an absence of aggregation). Adding 2mM lactose does not block aggregation (i.e., the loss in turbidity is just as extensive as in the control with nothing added). PMSF treatment of the dendrimer results in near-total abrogation of the dendrimer's inhibitory activity since the enzyme has no catalytic activity. This demonstrates that the failure to aggregate is dependent on the proteolytic activity of the glycodendrimer. Note also that 1.5 µg/ml (500nM) of S156C digal is more effective at blocking aggregation than is 15 µg/ml of the native enzyme and that 4.5 µg/ml S 156C-digal is more effective than 50 µg/ml native enzyme. In each case the 10-fold lower concentration of the glycodendrimeric protease yielded less aggregation that the native enzyme at a 10x higher concentration. At 50 µg/ml of S156C-digal (∼1.5µM) we see almost complete elimination of aggregation, the same level seen in the presence of 100mM lactose;

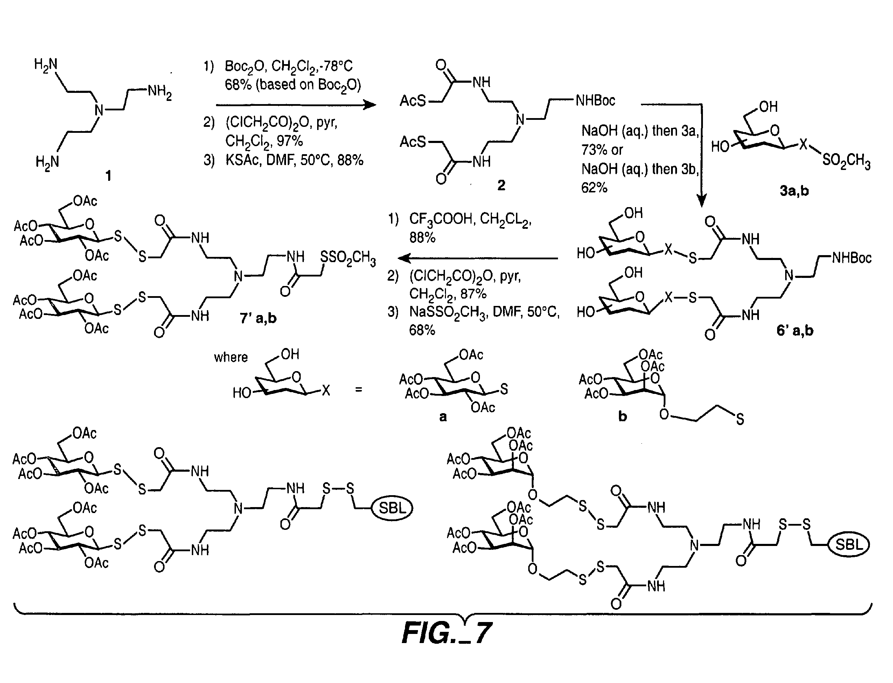

Figure 7 shows a normal addition synthetic scheme for producing two different first-generation **Type A (TREN-type)** glycodendrimer reagents, and glycodendriproteins produced from these reagents;

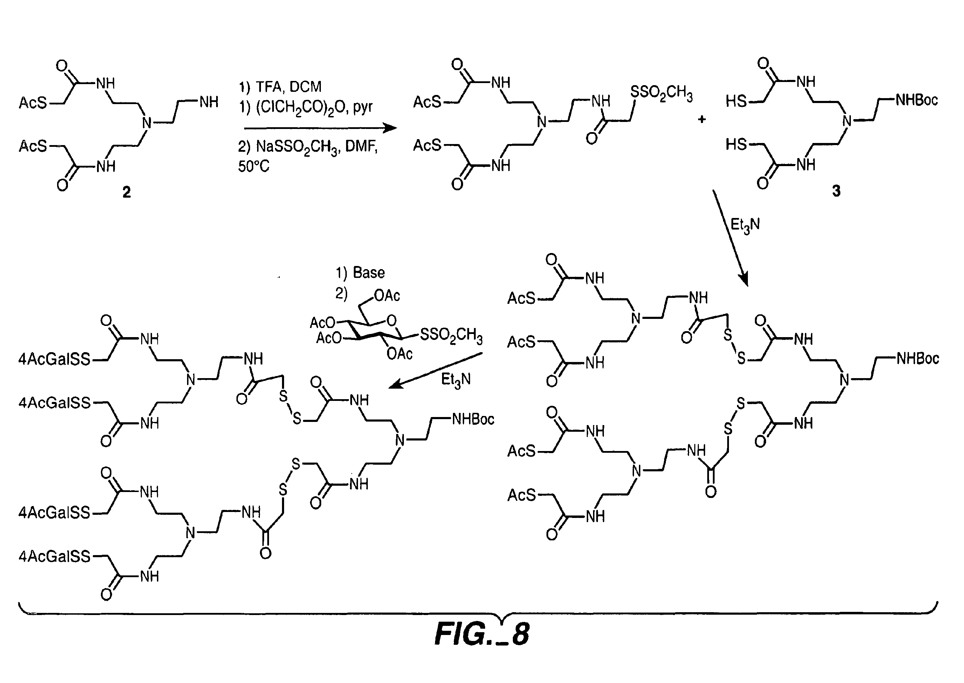

Figure 8 shows a normal addition synthetic scheme for producing a second generation Type A glycodendrimer reagent;

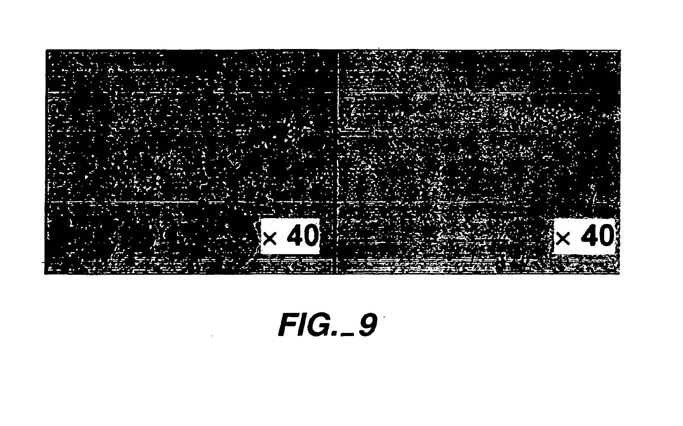

Figure 9 shows a visualization of reduced aggregation. Samples of the control aggregation reaction and the reaction containing 15 µg/ml S 156C-digal were transferred to glass slides for microscopic examination. The photmicrographs shown in Figure 9 confirm that decreased turbidity indeed correlates with decreased aggregation. A control is pictured on the left. It displays large aggregates of the two bacterial species. The reaction on the right was that in which *A. naeslundii* was incubated with the biantennary dendrimer (15 µg/ml) before *S*. *oralis* was added. It is clear that the dendrimer could significantly reduce the accumulation of bacteria on surfaces that were already colonized with one of the coaggregating partners.

### Detailed Description of the Preferred Embodiments

Unless defined otherwise herein, all technical and scientific terms used herein have the same meaning as commonly understood by one of ordinary skill in the art to which this invention belongs. Singleton, et al., DICTIONARY OF MICROBIOLOGY AND MOLECULAR BIOLOGY, 2D ED., John Wiley and Sons, New York (1994), and Hale & Marham, THE HARPER COLLINS DICTIONARY OF BIOLOGY, Harper Perennial, NY (1991) provide one of skill with a general dictionary of many of the terms used in this invention. Although any methods and materials similar or equivalent to those described herein can be used in the practice or testing of the present invention, the preferred methods and materials are described. Numeric ranges are inclusive of the numbers defining the range. Unless otherwise indicated, nucleic acids are written left to right in 5' to 3' orientation; amino acid sequences are written left to right in amino to carboxy orientation, respectively. Practitioners are particularly directed to Sambrook *et al.,* 1989, and Ausubel FM *et al.,* 1993, for definitions and terms of the art. It is to be understood that this invention is not limited to the particular methodology, protocols, and reagents described, as these may vary.

The headings provided herein are not limitations of the various aspects or embodiments of the invention which can be had by reference to the specification as a whole. Accordingly, the terms defined immediately below are more fully defined by reference to the specification as a whole

### Definitions

As used herein, a "glycodenrimeric protease" is an enzyme attached via disulfide linkage to a free thiol of a cysteine residue at one or more pre-seleted sites. The glycodendrimeric protease may have a branched or multiply-branched structure that presents a carbohydrate moiety at each branch extremity. Carbohydrate moieties may be selected from the group consisting of glucose, galactose, fucose, mannose, sialic acid, N-Acetylgalactosamine, N-Acetylglucosamine, and glucuronic acid. Useful glycodendrimeric proteases of the invention are shown in Figure 2. Useful examples can also be found, for example, in WO 02/079394.

Such an enzyme preferentially modifies an adhesin on a microorganism, such as a protein, by binding to the adhesin followed by proteolytic degradation of the adhesin. For example, such an enzyme can catalyze a reaction for modifying a molecule on the microorganism. An enzyme employed in a method or composition of the invention can, for example, catalyze a degradation of the binding site of an adhesin, such as a lectin or another carbohydrate binding site, on the microorganism. Preferably, the enzyme has one or more branched dendric antennae presented.

As used herein, "microorganism" refers to microbes including a eukaryote, a prokaryote or a virus and including, but not limited to, a bacterium (either gram positive, gram negative or gram-variable), a fungus, a virus, a protozoan and other microbes or microscopic organisms.

As used herein, "adhesin molecule" or "adhesin" means a molecule or complex of molecules that is typically expressed on the surface of a microorganism and that mediates adhesion by the microorganism to cells, tissues, extracellular matrix, teeth, a dental prosthesis, a medical device or catheter or another surface. Some adhesin molecules bind to a receptor on the surface of the other cell, tissue or extracellular matrix. Some adhesin molecules adhere to polysaccharides that coat teeth, gums, dental prostheses and the other tissues in the oral cavity. Some adhesin molecules adhere to polysaccharides or other molecules that coat body cavities, and tissues in these cavities, including the middle ear, vagina and the like or to other microorganisms that infect these cavities. Adhesins include carbohydrate binding proteins or sites on the surface of microorganisms. Adhesin molecules can be found in, on or as parts of lectins, proteins, enzymes, such as glucosyltransferases, hydrophobins, outer membrane proteins, flagella, fimbriae, pili, fibrillae and the like.

Microorganisms can adhere to carbohydrate portions of extracellular matrix (glyco)proteins; alternatively, bacteria can attach to protein portions of extracellular matrix molecules via microbial carbohydrates. A prominent example of this phenomenon is the interaction of the hyaluronic acid capsular polysaccharide of *Streptococcus pyogenes* with CD44 (protein) molecules on human cells (Courtney). Also, many conserved carbohydrate-containing bacterial components such as lipopolysaccharide and peptidoglycan (all displaying PAMPs - pathogen associated molecular patterns) have been found to bind to toll-like-receptors (TLR's) on a variety of mammalian cells (Kirschning). A third example is the colonization of teeth by *S. mutans,* which is in part mediated by the bacterium's association with tooth-anchored glucosytransferases via glucan bound to its surface.

As used herein, TREN refers to tris (2-aminoethyl)amine.

As used herein, "adhesion by a microorganism" refers to the binding of a microorganism to a cell, tissue, extracellular matrix, a tooth, a dental prosthesis or another surface, including hard surfaces that are cleaned by detergents or cleaners. The surface can be of a body cavity such as the oral cavity, vagina, middle ear or the like.

As used herein, "reduce adhesion by a microorganism" or "reducing adhesion by a microorganism" refers to decreasing the amount of adhesion by the microorganism to a cell, tissue, extracellular matrix, a tooth and/or dental prosthesis or to any other surface onto which microorganisms adhere and colonize. The decrease in adhesion can be observed by employing comparison to a control cell, tissue, extracellular matrix, a tooth and/or dental prosthesis or to a control population. Generally, "reduce" or "reducing" can also be expressed as inhibit or inhibiting, diminish or diminishing, abolish or abolishing and like terms. Reduction in adhesion by a microorganism by an amount that is measurable with statistical significance as less than a control value for adhesion by the microorganism can be expressed as "significantly reduced adhesion by a microorganism". Significant reduction in adhesion by a microorganism can also be determined by demonstrating a desired biological effect upon treatment of a microorganism with a glycodendrimeric protease, preferably including correlation of this effect with adhesion by the microorganism.

As used herein, "dental prosthesis" refers to a replacement for one or more of a mammal's teeth or another oral structure, including replacement of a single tooth, any type of denture and any type of bridge. A dental prosthesis can be either fixed in the mammal's oral cavity or removable from the mammal's oral cavity. As used herein, "denture" refers to any type of denture including a partial denture, a complete denture, a fixed denture and a removable denture.

As used herein "surface" refers to any surface to which a microorganism can bind or adhere. Surfaces include cells, tissues or extracellular matrix, among others. Surfaces also include the surface of any catheter, implant, prosthesis or other man made device that resides or is placed in or on a mammal's body or body cavity. Surfaces also include other surfaces to which a microorganism might bind such as a surface of a medical device external to the mammal, but that contacts the mammal or mammalian fluids or tissues, such as a periodontal dialysis apparatus, kidney dialysis apparatus, heart/lung machines and the like. Surfaces also include surfaces in other apparatus or equipment to which microorganisms can adhere, such as in brewing apparatus, fermentation apparatus, effluent treatment apparatus and other reactors and apparatus. Surfaces include hard surfaces that are cleaned by detergents or other cleaners.

As used herein, the terms "treating", "treatment" and "therapy" refer to curative therapy, prophylactic therapy and preventative therapy. Treating, treatment and therapy can reduce or ameliorate the severity or presence of symptoms of a disorder, can reduce or ameliorate the severity or presence of a disorder or can cure the disorder.

As used herein, the term "mammal" refers to any mammal classified as an animal, including humans, cows, horses, dogs and cats. In a preferred embodiment of the invention, the mammal is a human.

As used herein, the term "animal" refers to vertebrate animals including birds, mammals, reptiles, amphibians and the like. Preferred animals include mammals and birds.

As used herein, the term "composition" refers to substances that can be administered to a subject, preferably a mammal, to treat a disorder that may benefit from administering an enzyme, such as a glycodendrimeric protease, to reduce adhesion by one or more microorganisms.

As used herein, the term "oral care composition" refers to a composition suitable for administration to the oral cavity of a subject, preferably a mammal, to treat a disorder of or in the oral cavity that may benefit from administering an enzyme, such as a glycodendrimeric protease, to reduce adhesion by one or more microorganisms.

As used herein, the term "effective amount" refers to an amount of a glycodendrimeric protease sufficient to reduce or inhibit adhesion by a microorganism to a cell, tissue, extracellular matrix, a tooth, dental prosthesis or another surface, including hard surfaces that are cleaned by detergents or cleaners.

As used herein, the term "glycodendrimer" refers to chemically synthesized branching structures bearing carbohydrates on their branch tips. Glycodendrimers can be found, for example, in WO 02/079394.

As used herein, the terms "highly branched molecules" or "dendrimers" or "well-defined branching dendrimers" are synonymous and refer to structures of the invention as herein described. Highly branched molecules were first were synthesized by Vögtle in 1978 (Buhleier et al., Synthesis, 155-158 (1978)). The attachment of identical building blocks that contain branching sites to a central core may be achieved with a high degree of homogeneity and control. Each branch contains a functional group which, after chemical alteration, may be connected to yet another branching building block. In this manner, layer after layer of branching rapidly generates highly-functionalized molecules.

For instance, multiple glycosylation, including multiple mannose-containing chemically modified mutant proteins, and varied sugar moieties, can be created. The dendrimer reagent structures would include methanethiosulfonates with simple branching such as: 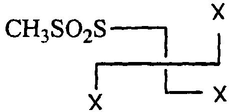 derived from pentaerythritol (i.e., "Penta-E"), to very complex branched dendrimer reagents (e.g., *see* Figure 1). In particular, a first generation glycodendrimer reagent is synthesized as shown in Figure 7. This approach can be extended to cover larger dendrimers. More specifically, by leaving one "arm" of the glycodendrimer free for conversion to a methanethiosulfonate, the remaining arms can be further branched to synthesize highly-functionalized glycodendrimer reagents as shown in Figure 8.

Glycodendrimeric proteases are employed to degrade the adhesin, and the enzyme can be modified. Preferred enzymatic modifications employ glycodendrimeric proteases having an appropriate branched structure to optimally present an isomeric form of the carbohydrate moieties selectively recognized by a particular adhesin or lectin . It is believed that the glycodendrimeric protease can act to inhibit cellular adhesion be selectively degrading the adhesion recognition site for attachment to specific cell surface glycoproteins on the host cell surface. Blocking this adhesion prevents a series of events including a tight binding event that can lead to infection and disease. In another scenario, tight binding can lead to the synthesis of components that will facilitate forming supportive biofilms. It is believed that by blocking the adhesion step by eliminating the adhesin recognition sites that the glycodendrimer functions as an anti-infective agent. It is believed that this glycodendrimer anti-infective agent could be effective alone or in combination with other antibiotics to enhance the antibiotic's function by prevention of attachment and/or the formation of biofilms which are believed to render the bacteria more resistant to antibiotic microbials. The selectivity of the glycodendrimer proteases may also have additional beneficial properties by targeting harmful bacterial while leaving innocuous and benevolent bacterial unaffected. The growth of innocuous bacterial is in itself potentially beneficial by virtue that these bacterial will compete for available nutrients with harmful bacteria thereby further limiting their growth.

### Methods and Compositions

Described herein are methods and compositions employing an enzyme, such as a glycodendrmeric protease, for reducing adhesion by a microorganism, preferably without killing or halting the growth of the microorganism. The methods and compositions can reduce or inhibit binding or adhesion of a microorganism to a cell, tissue or other surface. Further, the glycodendrimer, comprised of the same carbohydrate recognized by the bacterial adhesin, specifically directs the enzyme to bind at the site which would ordinarily be used by the bacterium to bind the target receptor, such as a carbohydrate, and therefore can be effective in much lower concentrations and have many fewer global effects on the bacterial cell and/or body site in which it is employed. The methods include administering effective amounts of the enzyme, for reducing or preventing adhesion by a microorganism, for example, at the site of an infection by a microorganism in an animal's body, including a body cavity, a dental prosthesis, a tissue, a site of catheterization or the like. The compositions include effective amounts of a glycodendrimeric protease, in a carrier suitable for maintaining this enzyme in a form active for reducing adhesion by a microorganism. In one embodiment, the composition includes a pharmaceutical composition, suitable for therapeutic administration to an animal. The compositions also include oral care compositions. The compositions also include compositions suitable for applying an enzyme such as a glycodendrimeric protease to a surface of a prosthesis, medical device (e.g. a catheter), a polymeric surface, a metal surface, or the like that can be cleaned with a cleaner or disinfectant.

The enzyme employed in the methods or compositions preferentially modifies an adhesin, such as a carbohydrate binding site, on the microorganism. Such an enzyme can catalyze a reaction for modifying an adhesin or other molecule on the microorganism or in the binding site of a lectin or another carbohydrate binding site on the microorganism. Adhesion by a microorganism can occur through a variety of mechanisms to a variety of substrata. For example, microorganisms that inhabit an animal's oral cavity can adhere to polysaccharides that coat teeth, gums, tongue, throat, cheeks, a dental prosthesis and the other tissues in the oral cavity. Microorganisms also can adhere to carbohydrates on cells, tissues and extracellular matrix in or on the animal's body or in or on one of the animal's body cavities. The cells can include other microorganisms. Such microorganism to microorganism binding can be referred to as coaggregation. Microorganisms that coaggregate include microorganisms that are early and late colonizers of freshly cleaned teeth. Microorganisms can also adhere (less frequently) to proteins on cells, tissues, and extracellular matrix via microbial carbohydrate residues. In this case, the enzyme employed in the methods or compositions preferentially modifies a camohydrate-binding residue on the host cell.

Microorganisms frequently employ adhesins, including carbohydrate binding structures such as lectins and glucosyltransferases. Microorganisms that employ adhesin molecules include bacteria, such as *Actinobacillus actinomycetemcomitans, Actinomyces israelii, A. naeslundii and A. viscosus, Capnocytophaga ochracea, Eikenella corrodens, Escherichia coli, Fusobacterium nucleatum, Haemophilus influenzae, Porphyromonas gingivalis, Prevotella intermedia, Proteus mirabilis, Proteus vulgaris, Pseudomonas aeruginosa, P. loeschei, Streptococcus gordonii, S. mutans, S. oralis, S sanguis,* various group A streptococci, various invasive and antibiotic resistant staphylococci, and *Treponema denticola;* viruses such as influenza virus; yeasts, such as *Candida albicans;* and protozoans, such as *Entamoeba histolytica.* Adhesin molecules of several M5, M6 and M24 positive strains of streptococci have been studied (Dale J.B. et al., Vaccine 14-944-948 (1996); Courtney et al., FEMS Microbiol. Letters 151:65-70 (1997)). *P. aeruginosa* makes a good model for study as its adhesion can depend on two lectins, PA-1 and PA-2. Furthermore, the bacterium will form biofilms on a variety of surfaces, ranging from glass and steel to human lungs.

Adhesion by a microorganism can be determined by employing a variety of techniques known to those of skill in the art. These techniques include determining coaggregation of the microorganism of interest with a cell, such as through turbidimetry, determining aggregation of microorganisms with a polysaccharide, such as formation of an aggregate in solution or a pellicle, determining binding of the microorganism to a mammalian cell, monitoring hemagglutination and determining binding of a microorganism to extracellular matrix.

Typical assays for aggregation or coaggregation including microorganisms can be done in a suitable buffer and can involve visual end-point estimates or kinetic measurements, such as those employing a platelet aggregometer. Visual end-points can be determined by methods known to those of skill in the art, such as those described by Kolenbrander (Kolenbrander, P.E., Meth. Enzymol. 253:385-396 (1995)). A more quantitative method for measuring coaggregation involves mixing the two coaggregating partners together in a test tube and observing the decrease in the optical density of the mixture over time. As aggregates form they settle to the bottom of the tube and the rate and extent of settling can be measured by the decrease in optical density. The decrease is measured continuously in a spectrophotometer and plotted on a graph of optical density vs. time.

Suitable reaction pairs of microorganisms for measuring coaggregation include:

| Contains adhesin molecule: | Contains receptor: | Control Reaction can Employ Protection by: |
|---|---|---|
| | | |
| *A. naeslundii* T14V | *S. oralis 34* | Lactose |
| *Capno*cy*tophaga ochracea* | *A. viscosus* | Rhamnose |
| *F. nucleatum* | *A. israelii* | Lactose |
| *F. nucleatum* | *A. actinomycetemcomitans* | Unknown |
| *Prevotella intermedia 27* | *A. naeslundii* | None |
| *Prevotella loeschei* | *S. oralis* | Lactose |
| *S. gordonii* | *A. naeslundii* | None |
| *S. sanguis* | *Porphyromonas gingivalis W50* | None |

Typically, in these pairs of microorganisms, the adhesin molecule is inactivated by heat and/or pronase, indicating that it is a protein, but the receptor is resistant to heat and/or pronase, meaning it is a non-protein, typically, a carbohydrate. These microbes represent early and late colonizers, Gram-positive and Gram-negative, those susceptible to protection by carbohydrates and those resistant to effects of carbohydrates. Nearly always one of the bacterial partners uses a protein to bind to a carbohydrate on its partner bacterium, a type of reaction particularly amenable the current invention. Additional coaggregating pairs have been employed in studies reported, as described in the Figures and Examples provided herein.

Adhesion of a microorganism to a cell from an animal tissue can be determined by any of a variety of methods known to those of skill in the art. Such methods include, for example, assaying *E. coli* strains possessing Pap-type fimbriae for adhesion to their substratum, di-galactose, by mixing them with suspensions of latex beads conjugated with the disaccharide (EY Laboratories) according to the procedure of Garcia *et al.* (Garcia E. et al., Curr. Microbiol. 17:333-337(1988)). For adhesion of the group A streptococci, human laryngeal cells (HEp-2 from ATCC) or sloughed buccal epithelial cells can be used as substrata. For such assays, bacteria can be tested at a variety of densities, starting, for example, at a high of 10⁹/ml with dilutions down to about 10⁷/ml or lower. These ranges can be used to generate a binding isotherm as described in Chapter 2 of Ofek and Doyle, 1994 *supra.* The adhesion reaction mixture can be incubated then aspirated and washed with medium to remove non-adherent or adventitiously bound cells. The data obtained may yield, for example, regular binding isotherms, Langmuir plots, Scatchard plots and/or analysis of "cooperative" adhesion (Ofek and Doyle, *supra).* For example, if the glycodedrimeric protease of the present invention abolish a positive slope of a Scatchard plot of adhesion results, it could be said the enzyme is preventing positive cooperativity.

*C. albicans is* a fungal microorganism that can infect denture wearers, head-neck irradiated patients, Sjögren's patients, AIDS patients, and other immunocompromised subjects. Adhesion of *C. albicans* to various substrata can be determined employing the general procedures of Hazen and Glee (Hazen, K.C. and Glee, P.M., Meth. Enzymol. 253:414-424 (1995)) and of Segal and Sandovsky-Losica (Segal, E. and Sandovsky-Losica, H., Meth. Enzymol. 253:439-452 (1995)). *C. albicans* for adhesion studies can be obtained from exponential (yeast phase) cultures in YE (yeast extract).

A variety of microorganisms, such as oral bacteria, can adhere to many substrata including various extracellular matrix proteins. Adhesion to extracellular matrix proteins can be measured by a variety of methods known to those of skill in the art. For several oral bacteria, fibronectin is a receptor for their adhesin molecules. For others, collagen serves as a receptor. Other receptors are also known. (Ljungh, A. and Wadström, T. Meth. Enzymol. 253:501-573 (1995)). Bacteria known to adhere to collagen include *Actinomyces viscosus, Porphyromonas gingivalis,* and *Prevotella intermedia. (see,* for example, Liu, T., R.J. Gibbons, D.I. Hay, and Z. Skobe, Oral Microbiol Immunol. 6:1-5 (1991); Naito, Y., and R.J. Gibbons, J. Dent. Res. 67:1075-1080; Grenier, D., include *S. sanguis, S. pyogenes* M5⁺ protein and *Treponema denticola* (reviewed in Ofek & Doyle, Bacterial Adhesion to Cells and Tissues. Chapman and Hall, New York. 1994). Adhesion to collagen can be studied by the method described by Grenier 1996 supra; and experiments with fibronectin can be patterned after those with collagen. Microorganisms can adhere to carbohydrate portions of extracellular matrix (glyco)proteins; alternatively, bacteria can attach to protein portions of extracellular matrix molecules via microbial carbohydrates and/or proteins.

The microorganisms employed in studies of adhesion can be produced and isolated by any of a variety of methods known to those of skill in the art. For example, microorganisms can be purchased, obtained from clinical isolates, or prepared in other ways. Protozoa such as *E*. *histolytica* can be grown axenically as described by Petri and Schnaar (Petri, W.A. Jr. and Schnaar, R.L., Meth. Enzymol. 253:98-104 (1995)). The trophozoites can be harvested and washed as described. Adhesion studies can employ trophozoite membranes and hemagglutination to assay for the lectin. Viruses, such as influenza A virus, can be cultured, harvested, and handled according to procedures well known in the art.

Administering an effective amount of an enzyme, such as a glycodendrimeric protease, to animal tissues, cells, extracellular matrix, teeth and/or dental prosthesis preferably results in a decrease in adhesion by one or more microorganisms sufficient to ameliorate detrimental effects or disease resulting from such adhesion. Effective administration or use of the enzyme, such as a glycodendrimeric protease, in this manner is typically evidenced by prevention or inhibition of infection, reduction or moderation of symptoms of an infection, reduction of adhesion, and the like. Absence or reduction of infection and moderation of symptoms can be determined by common clinical or laboratory methods. Reduction of adhesion can be determined by plate counts, microscopy, aggregometry, turbidimetry, isotopic labeling and other methods standard in the art.

An enzyme, such as a glycodendrimeric protease, that decreases adhesion can be useful in one or more of a variety of applications including: fighting biofouling, for example in peritoneal dialysis; reducing dental caries; treating symptoms of infection by reducing adhesion of *E. coli* in an animal's gut; treating infection by reducing adhesion by one or more protozoa, such as *Entamoeba;* treating ulcers, for example by reducing adhesion of *Helicobacter;* treating viral infections by reducing adhesion of viruses, such as influenza virus; serving as a birth control agent, since sperm and egg interact via similar lectin-mediated interactions as described for microorganisms; reducing contamination of eggs and/or other poultry products by serving as a chicken feed supplement for reducing levels in the bird of salmonellae; treating infection of periodontal tissue, eye, ear, or throat, such as by reducing adhesion by *Haemophilus, Streptococcus,* or *Candida;* as a component of eye or ear drops, of a gargle (e.g. for sore throat), of a gels in a periodontal disease packing; killing mosquito larvae when cloned into Bt; as a probiotics (e.g. by cloning glycodrimeric protease into *Lactobacillus);* or for fighting skin infections (impetigo) caused by *Staphylococcus* or *Streptococcus* or *Vibrio cholerae* infections (caused by *Vibrio* toxin binding to CHO) and other bacterial diseases which rely on toxin binding to host tissues, such as whooping cough and diphtheria.

The methods and compositions described herein can be employed to treat urinary tract infections. Such infections are responsible for 9.6 million physician visits per year. The vast majority of these are caused by *E. coli* possessing various fimbrial structures. Nearly all *E. coli* strains express type 1 fimbriae, and certain allelic variants of the fimbriae are associated with the ability to colonize the lower urinary tract, utilizing mannose on host cells for attachment. P-fimbriated *E*. *coli* use digalactose structures for attachment and are strongly associated with upper urinary tract (i.e., kidney) infections.

The methods and compositions described herein can be employed to treat infections at sites of catheters and/or cannulas. Organisms such as *Proteus mirabilis, Proteus vulgaris* and *Pseudomonas aeruginosa* frequently colonize catheters, resulting in catheter removal and/or infection in the subject.. Adhesion can lead to encrustation because the ammonia from urease will increase pH enough to precipitate struvite (Mg-NH₄-phosphate) and hydroxylapatite (Ca phosphate). It may be that a glycodendrimeric protease can inhibit urease and/or adhesion. Or it may be that the enzymes reduce adhesion but not have any effects on urease. In either case, it is desired to reduce encrustation and prolong the life of the catheter. A model provided in some detail by Tunney et al. (1999, Biofilm and biofilm-related encrustation of urinary tract devices. Meth. Enzymol. 310:558-566) can be employed to demonstrate the effectiveness of an enzyme such as a glycodendrimeric protease against such adhesion related encrustation. Catheters and like instruments can be coated or otherwise treated with an enzyme, such as a glycodendrimeric protease to reduce or delay adhesion and/or encrustation.

The methods and compositions described herein can be employed to treat infections of body cavities, including the vagina and the middle ear. By treating infections of the vagina, the methods and compositions can also treat infections of newborns. Group B streptococcus is the most common cause of life threatening infections in newborns. The infection is acquired by infants during passage through the birth canal and also during the post-partum period. Reducing adhesion of these microorganisms to the newborn or to the vagina can reduce or treat such infections. *Streptococcus pneumoniae* and *Haemophilus influenzae* are the #1 and #2 cause of middle ear infections (otitis media). Disrupting adhesion by these bacteria to epithelial or other cells of the ear can reduce or treat such infections.

The methods and compositions described herein can be employed to treat infections of nonhuman animals, such as birds, particularly chickens. Compositions of the present inventions include compositions suitable for veterinary use. Treatment of animals used for meat or dairy products can be employed to prevent or reduce the incidence of food borne illnesses. For example, *Salmonella*-contaminated eggs have been implicated more than any other source as causing food borne illness. Chicks that acquire *S. enteritidis* have the bacterium for life, leading to egg contamination. Disrupting adhesion by these bacteria to cells of the digestive or egg producing tracts of the chicks can treat such infections. An enzyme that reduces adhesion of a microorganism can be administered to the chick or other food producing animal in water, food, or by other suitable methods. Enzyme administered through food or water is preferably stable in the digestive tract, such as an enteric composition or a stabilized recombinant variant of the enzyme.

The methods and compositions described herein can also be employed against adhesion of microorganisms to synthetic surfaces, such as those of prostheses, of catheters or cannulas, of other medical devices or equipment, or of apparatus employed in brewing, fermentation, effluent treatment, and the like. Enzymes are commonly employed in cleaning or sanitizing compositions. Enzymes that reduce adhesion by microorganisms, such as a glycodendrimeric protease, can be formulated by methods and in formulations known to those of skill in the art for inclusion in cleaning and/or sanitizing compositions. In certain circumstances such enzymes can be employed during the process or treatment effected by the device or apparatus to reduce adhesion of microorganisms.

### Pharmaceutical Compositions

Described herein are pharmaceutical compositions including an enzyme, such as a glycodendrimeric protease. An enzyme, such as a glycodendrimeric protease, can be used in such pharmaceutical compositions, for example, for the treatment of microorganismal pathologies. It is contemplated that the pharmaceutical compositions of the present invention can be used to treat infections by one or more microorganisms that rely upon an adhesin molecule with a binding site.

The pharmaceutical compositions described herein preferably contain an effective amount of an enzyme, such as a glycodendrimeric protease, to reduce adhesion by a microorganism. Optionally, the pharmaceutical composition may include agent(s) that stabilize or augment the activity of a glycodendrimeric protease. Such agents include, but are not limited to, starch, gelatin, carrageenan, glycols and other agents used to compound pharmaceuticals.

The pharmaceutical compositions described herein include an enzyme, such as a glycodendrimeric protease, in a pharmaceutically acceptable carrier. Pharmaceutically acceptable carriers are known to those skilled in the art and include materials useful for the purpose of administering a medicament, which are preferably non-toxic, and can be solid, liquid, or gaseous materials, which are otherwise inert and medically acceptable and are compatible with the enzyme, such as a glycodendrimeric protease, and any other active ingredient that is present.

Water, saline, aqueous dextrose, and glycols are preferred liquid carriers, particularly when isotonic, for injectable solutions. The carrier can be selected from various oils, including those of petroleum, mammal, vegetable or synthetic origin, for example, peanut oil, soybean oil, mineral oil, and sesame oil. Suitable pharmaceutical excipients include starch, cellulose, talc, glucose, lactose, sucrose, gelatin, malt, rice, flour, chalk, silica gel, magnesium stearate, sodium stearate, glycerol monostearate, sodium chloride, dried skim milk, glycerol, propylene glycol, water, and ethanol. The compositions can be subjected to conventional pharmaceutical expedients, such as sterilization, and can contain conventional pharmaceutical additives, such as preservatives, stabilizing agents, wetting, or emulsifying agents, aerosolizing agents, salts for adjusting osmotic pressure, or buffers. Suitable pharmaceutical carriers and their formulations are described in Martin, "Remington's Pharmaceutical Sciences," 15th Ed.; Mack Publishing Co., Easton (1975); see, e.g., pp. 1405-1412 and pp. 1461-1487. Such compositions will, in general, contain an effective amount of an enzyme, such as a glycodendrimeric protease, to reduce adhesion by a microorganism, together with a suitable amount of carrier so as to prepare the proper dosage form for proper administration to the animal.

The pharmaceutical compositions described herein can be administered by various routes, including orally, used as a suppository or pessary; applied topically as an ointment, cream, aerosol, powder; or given as eye or nose drops, etc., depending on whether the preparation is used to treat internal or external infections by one or more microorganisms. The compositions can contain 0.1% - 99% of the enzyme, such as a glycodendrimeric protease. Preferably, the composition includes about 0.1 wt-% to about 1.0 wt-% of an enzyme, such as a glycodendrimeric protease. The enzymes are usually soluble in pharmaceutical preparations.

For oral administration, fine powders or granules can contain diluting, dispersing and/or surface active agents, and can be presented in a draught, in water or in a syrup; in capsules or sacnets in the dry state or in a non-aqueous solution or suspension, wherein suspending agents can be included; in tablets or enteric coated pills, wherein binders and lubricants can be included; or in a suspension in water or a syrup. In some cases, the enzyme(s) may be formulated to form aerosols. Where desirable or necessary, flavoring, preserving, suspending, thickening, or emulsifying agents can be included. Tablets and granules are preferred, and these can be coated. A preferred formulation for oral administration includes agents that maintain the activity of an enzyme, such as a glycodendrimeric protease, in the stomach and intestines. Such agents include buffers and "slow release" components.

For buccal administration, the compositions can take the form of tablets or lozenges formulated in a conventional manner.

Alternatively, for infections of the skin or other external tissues the compositions are preferably applied to the infected part of the body of the animal as a topical ointment, cream or spray. The enzyme, such as a glycodendrimeric protease, can be presented in an ointment, for instance with a water-soluble ointment base, or in a cream, for instance with an oil in water cream base. Carriers for topical or gel-based forms of include polysaccharides such as methylcellulose; polyvinylpyrrolidone, polyacrylates, polyoxyethylene-polyoxypropylene-block polymers, polyethylene glycol, and wood wax alcohols. For topical administration, an enzyme, such as a glycodendrimeric protease, can be present in the pharmaceutical composition in a concentration of from about 0.01 to 10%, preferably 0.1 to 1.0% w/v. For topical administration, the daily dosage as employed for adult human treatment will range from 0.1 mg to 1000 mg, preferably 0.5 mg to 10 mg. However, it will be appreciated that extensive skin infections can require the use of higher doses.

For all administrations, conventional depot forms are suitably used. Such forms include, for example, microcapsules, nano-capsules, liposomes, plasters, inhalation forms, nose sprays, sublingual tablets, and sustained-release preparations. The enzyme, such as a glycodendrimeric protease, will typically be formulated in such carriers at a concentration of about 0.1 mg/ml to 100 mg/ml. An enzyme, such as a glycodendrimeric protease, can also be administered in the form of a sustained-release preparation. Suitable examples of sustained-release preparations include semipermeable matrices of solid hydrophobic polymers containing the protein, which matrices are in the form of shaped articles, e.g., films, or microcapsules. Examples of sustained-release matrices that are well known in the art include polyesters, hydrogels, polylactides, copolymers of L-glutamic acid and gamma ethyl-L-glutamate, non-degradable ethylene-vinyl acetate, degradable lactic acid-glycolic acid copolymers, and poly-D-(-)-3-hydroxybutyric acid.

An enzyme, such as a glycodendrimeric protease, can also be administered employing a composition suitable for gene therapy. For *in vivo* delivery of a nucleic acid (optionally contained in a vector) into an animal's cells, the nucleic acid is injected directly into the animal, usually at the sites where the polypeptide is required. Known *in vivo* nucleic acid transfer techniques include transfection with viral or non-viral vectors (such as adenovirus, lentivirus, Herpes simplex I virus, or adeno-associated virus (AAV)) and lipid-based systems (useful lipids for lipid-mediated transfer of the gene are, for example, DOTMA, DOPE, and DC-Chol; see, e.g., Tonkinson et al., Cancer Investigation, 14(1)): 54-65 (1996)). A viral vector typically includes at least one element that controls gene expression, an element that acts as a translation initiation sequence, a packaging signal, long terminal repeats (LTRs) or portions thereof, and positive and negative strand primer binding sites appropriate to the virus used (if these are not already present in the viral vector). In addition, such vector typically includes a signal sequence for secretion of an enzyme, such as a glycodendrimeric protease, from a host cell in which it is produced.

### Oral Care Compositions

Described herein are oral care compositions including an enzyme, such as a glycodendrimeric protease. An enzyme, such as a glycodendrimeric protease, can be used in such oral care compositions, for example, for the treatment of pathologies in which a microorganism infects the oral cavity. It contemplates that the oral care compositions of the present invention can be used to treat any infection by one or more microorganisms that rely upon an adhesin molecule.

The oral care compositions described herein preferably contain an effective amount of an enzyme, such as a glycodendrimeric protease, to reduce adhesion by a microorganism to cells or tissue of the oral cavity or to a dental prosthesis (e.g. a denture). The enzyme, such as a glycodendrimeric protease, is preferably resistant to pasteurization, stable in compounding agents and amenable to formulation as a solid, liquid or aerosol. Optionally, the oral care composition may include agent(s) that stabilize or augment the activity of the enzyme, such as a glycodendrimeric protease. Such agents include trace metals, such as copper ions, and oxygen generating compounds, such as hydrogen peroxide.

Oral care compositions including an enzyme, such as a glycodendrimeric protease, can be used, for instance; for maintaining and/or improving oral hygiene in the oral cavity of mammals, and/or preventing or treating dental diseases in mammals. The oral care compositions can also be used for reducing adhesion by one or more microorganisms to a dental prosthesis. For example, a denture can be cleaned with an enzyme, such as a glycodendrimeric protease, containing oral care composition either in the wearer's oral cavity or removed from the wearer's oral cavity. Oral care compositions include but are not limited to toothpaste, a dental cream, gel or tooth powder, a mouth wash or rinse, a denture cleaning agent (e.g. a cream or a soak), a chewing gum, a lozenge, and a candy. The oral care composition can be in the form of a solid, a semi-solid (e.g. a gel, a paste, or a viscid liquid), a liquid, or an aerosol.

Various ingredients that may be included in a tooth paste or gel and a mouth wash or rinse are well known in the art. In addition to an enzyme, such as a glycodendrimeric protease, a toothpaste or gel of the present invention will typically include one or more abrasives or polishing materials, foaming agents, flavoring agents, humectants, binders, thickeners, sweetening agents, or water. An enzyme, such as a glycodendrimeric protease, containing mouth wash or rinse will typically also include a water/alcohol solution and one or more flavors, humectants, sweeteners, foaming agents, and colorants.

Suitable, known abrasives or polishing materials include alumina and hydrates thereof (e.g. alpha alumina trihydrate), magnesium trisilicate, magnesium carbonate, sodium bicarbonate, kaolin, aluminosilicates (e.g. aluminum silicate), calcium carbonate, zirconium silicate, powdered plastics (e.g. powdered polyvinyl chloride, polyamide, or various resins) xerogels, hydrogels, aerogels, calcium pyrophosphate, water-insoluble alkali metaphosphates, dicalcium phosphate and/or its dihydrate, dicalcium orthophosphate, tricalcium phosphate, particulate hydroxylapatite, and mixtures of these abrasives or polishing materials. Typically, the abrasive or polishing material can be present in from 0 to about 75% by weight, preferably from 1% to about 65%, more preferably, for toothpastes or gels, about 10% to about 55% by weight of the toothpaste or gel.

Suitable, known humectants, which are typically employed to prevent loss of water from a toothpaste or gel, or other composition, include glycerol, polyol, sorbitol, polyethylene glycols (PEG), propylene glycol, 1,3-propanediol, 1,4-butane-diol, hydrogenated partially hydrolyzed polysaccharides, and mixtures of these humectants. In a toothpaste or gel, humectants are typically at about 0% to about 75%, preferably about 5 to about 55% by weight of the composition.

Suitable, known thickeners and binders, which maintain stability of an oral care composition include silica, starch, tragacanth gum, xanthan gum, extracts of Irish moss, alginates, pectin, certain cellulose derivatives (e.g. hydroxyethyl cellulose, carboxymethyl cellulose, or hydroxy-propyl cellulose), polyacrylic acid and its salts, and polyvinylpyrrolidone. Typically, a toothpaste or gel includes about 0.1 % to about 20% by weight of one or more thickeners and about 0.01 % to about 10% by weight of one or more binders.

A suitable foaming agent or surfactant in such oral care compositions will typically not significantly decrease the activity of an enzyme, such as a glycodendrimeric protease, present in the composition. Such foaming agents or surfactants can be selected from anionic, cationic, non-ionic, and amphoteric and/or zwitterionic surfactants. These can include fatty alcohol sulphates, salts of sulphonated mono-glycerides or fatty acids having 10 to 20 carbon atoms, fatty acid-albumin condensation compositions, salts of fatty acids amides, taurines, and/or salts of fatty acid esters of isothionic acid. The foaming agent or surfactant can be at levels in the composition from about 0% to about 15%, preferably from about 0.1 % to about 10%, more preferably from 0.25 to 7% by weight.

Suitable, known sweeteners include artificial sweeteners such as saccharin and aspartame. Suitable, known flavors include spearmint and peppermint. Such flavors or sweeteners are typically present at levels from about 0.01% to about 5% by weight, or from about 0.1% to about 5%.

The oral care compositions can also include one or more added antibacterials, anti-calculus agents, anti-plaque agents, compounds which can be used as fluoride source, dyes/colorants, preservatives, vitamins, pH-adjusting agents, anti-caries agents; or desensitizing agents.

An oral care composition including an enzyme, such as a glycodendrimeric protease, can be applied to the oral cavity of a mammal employing any of numerous methods known in the art for administering oral care compositions. For example, the oral care composition can be applied as any commonly applied toothpaste or mouthwash. The oral care composition can be introduced into the oral cavity, applied to an oral tissue, such as teeth and/or gums, removed from the oral cavity (e.g. by rinsing), and the oral cavity can be rinsed. Alternatively, the oral care composition can be applied to the periodontal pocket as a semi-solid or as a solid implant. A gel, paste, or viscid liquid can be applied with, for example, a toothbrush, a swab, a finger, a syringe, or a dentist's tool. In yet another embodiment, the oral care composition can used to soak a denture.

Yet another embodiment includes a composition suitable for reducing or inhibiting adhesion or binding of microorganisms to hard surfaces, including dental prostheses, medical devices, implants, counters, porcelain or plastic fixtures, instruments, and the like.

### Articles of Manufacture

Also described herein are articles of manufacture. An article of manufacture such as a kit containing an enzyme, such as a glycodendrimeric protease, useful for reducing adhesion by a microorganism, or for the treatment of the disorders described herein, includes at least a container and a label. Suitable containers include, for example, bottles, vials, syringes, and test tubes. The containers may be formed from a variety of materials such as glass or plastic. The container holds a composition that is effective for treating the condition and may have a sterile access port. The active agent in the composition is the enzyme, such as a glycodendrimeric protease. The label on, or associated with, the container indicates that the composition is used for treating the condition of choice. The article of manufacture may further include a second container including a pharmaceutically-acceptable buffer, such as phosphate-buffered saline, Ringer's solution, and dextrose solution. It may further include other materials desirable from a commercial and user standpoint, including other buffers, diluents, filters, needles, syringes, and package inserts with instructions for use. The article of manufacture may also include a second or third container with another active agent as described above.

The present invention may be better understood with reference to the following examples. These examples are intended to be representative of specific embodiments of the invention, and are not intended as limiting the scope of the invention.

### Example 1

### Competitive Enzyme-Linked Lectin Assays (ELLA).

All incubations were carried out at 37°C. Microtiter plates were coated overnight with peanut agglutinin (PNA) solution (100 ∝L of a 0.1mg/mL solution in phosphate buffered saline pH 7.2 (PBS)). Excess protein was removed by washing (3 · 200∝L of PBS containing 0.05% v/v Tween (PBST)). Coated wells were blocked with bovine serum albumin solution
(150 ∝L of a 1% w/v solution in PBS (PBST)) for 1h. During this period, serial (x2) dilutions of methyl β-D-galactopyranoside were incubated with glycodendriprotease (at a dilution of which had been determined to give A410 of 0.8-1.0 after 20 min). These solutions were then transferred to the blocked plate for an additional 1 h incubation. These plates were subsequently washed (2 · PBST, 1 · PBS) prior to development with suc-AAPF-*p*NA (50 ∝L of a 10 mM solution in 0.1M Tris, pH 8.6). The absorbance at 410 nm was monitored after a 20 min period. Binding constants are based on A410 in the absence of methyl β-D-galactopyranoside and referenced to the inhibitory potential of an methyl ζ-D-galactopyranoside determined for each plate and assuming a *K_{D}* = 5.34 10⁻⁴ .( G. B. Reddy, V. R. Srinivas, N. Ahmad, A. Surolia, *J*. *Biol. Chem.* **274***,* 4500-4503 (1999)) All assays were carried out in duplicate.

### Example 2

To assess the ability of these Gal-presenting synthetic glycoproteins we used an enzyme-linked lectin assay (ELLA)(7)(35) that utilized surface-immobilized lectin to mimic the display of surface lectins on bacterial surfaces and the inherent peptidase enzyme activity of SBL as a readout. These revealed a steady increase in affinity for the model Gal-binding lectin peanut agglutinin (PNA) with increasing numbers of Gal-antennae (*KD* for S156C-1, **2a, 2b, 3,4** ∼ 1.1 □ 10-3, 1.5 □ 10-3, 1.8 □ 10-3, 3.4 □10-4, 1.4 □ 10-7 M, respectively). Once such Gal-specfic lectin binding had been established, we evaluated the ability of these synthetic glycoproteins to inhibit the function of pathogens that depend on Gal-binding.

| Peanut Agglutinin Surface Binding Assay | |
|---|---|
| Glycodendrimeric protease | K_{D} |
| S156C-1‡ | 1.1*10⁻³ M |
| S156C-2a | 1.5*10⁻³ M |
| S156C-2b | 1.8*10⁻³ M |
| S156C-3 | 3.4*10⁻⁴ M |
| S156C-4 | 1.4*10⁻⁷M^{†} |

| | |
|---|---|
| ‡Designations correspond to structures shown in Figure 1. † Determined by linear extrapolation using various concentration of dendrineric protease and methyl β-D-galactopyranoside. | |

It can be seen the the affinity of binding to peanut agglutinin follows the number of saccharide moieties attach as sugar presenting antennae following what has been previously observed for surface based glycoprotein recognition by different adhesions.

It is understood that the examples and embodiments described herein are for illustrative purposes only and that various modifications or changes in light thereof will be suggested to persons skilled in the art, as defined by the appended claims .

## Claims

1. A glycodendrimeric protease, the glycodendrimeric protease having a branched or multiply-branched structure that presents a carbohydrate moiety at each branch extremity, wherein the glycodendrimer protease inhibits adhesion of one or more microorganisms to a surface,
wherein said glycodendrimer protease has the structure. 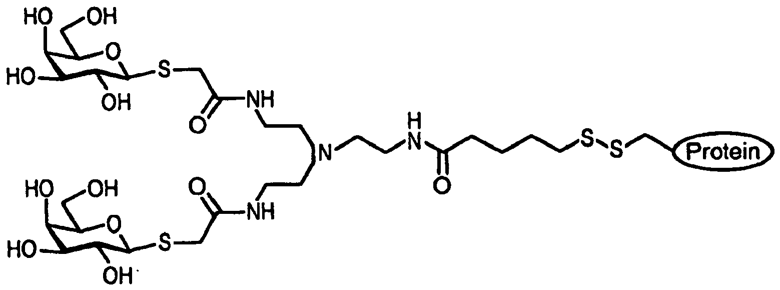 and wherein the protease is subtilisin *Bacillus lentus* S 156C.

2. The glycodendrimeric protease of claim 1, wherein the surface is selected from the group consisting of cells, tissues, extracellular matrix, teeth and prostheses.

3. The glycodendrimeric protease of claim 1, wherein the microorganism comprises a prokaryote, a eukaryote, a virus or a combination thereof.

4. The glycodendrimeric protease of claim 3, wherein the prokaryote is a gram-positive, gram-negative or gram-variable bacterium.

5. The glycodendrimeric protease of claim 4, wherein the prokaryote is an *Actinomyces* or *E. coli.*

6. The glycodendrimeric protease of claim 1, wherein the glycodendrimeric protease inhibits adhesion by binding to a lectin.

7. A composition comprising a glycodendrimeric protease according to any one of claims 1 to 6.

8. The composition of claim 7, which is an oral care composition.

9. The oral care composition of claim 8, wherein said oral care composition is a mouthwash or toothpaste.

10. The glycodendrimeric protease according to any one of claims 1 to 6, for use in a method of treatment of a disease in a patient in which it is desired to inhibit adhesion of one or more microorganisms to a surface of a body cavity.

11. Use of a glycodendrimeric protease according to any one of claims 1 to 6 in the preparation of a medicament for inhibiting adhesion of microorganisms to a surface.

12. Use according to claim 11, wherein the surface is selected from the group consisting of cells, tissues, extracellular matrix, teeth and prostheses.

13. Use according to claim 12, wherein the surface is mammalian oral cells or tissues, or a dental prosthesis.

14. Use according to claim 13, wherein the mammalian oral tissues or cells or dental prosthesis are those of a human.

15. Use according to claim 11, wherein said medicament is for topical administration.

## Patentansprüche

1. Glykodendrimer-Protease mit einer verzweigten oder mehrfach verzweigten Struktur, die am Ende jedes Zweiges eine Kohlenhydratgruppierung aufweist, worin die Glykodendrimer-Protease die Adhäsion eines oder mehrerer Mikroorganismen an einer Oberfläche hemmt,
worin die Glykodendrimer-Protease die nachstehende Struktur aufweist 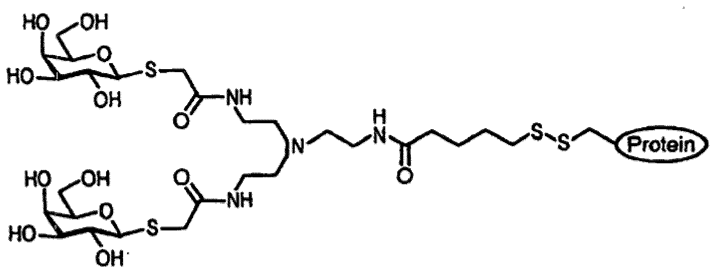 und worin die Protease Subtilisin *Bacillus lentus* S156C ist.

2. Glykodendrimer-Protease nach Anspruch 1, worin die Oberfläche aus der aus Zellen, Geweben, extrazellulärer Matrix, Zähnen und Prothesen bestehenden Gruppe ausgewählt ist.

3. Glykodendrimer-Protease nach Anspruch 1, worin der Mikroorganismus einen Prokaryoten, einen Eukaryoten, einen Virus oder eine Kombination daraus umfasst.

4. Glykodendrimer-Protease nach Anspruch 3, worin der Prokaryot ein grampositives, gramnegatives oder gramvariables Bakterium ist.

5. Glykodendrimer-Protease nach Anspruch 4, worin der Prokaryot ein *Actinomyces* oder *E. coli* ist.

6. Glykodendrimer-Protease nach Anspruch 1, worin die Glykodendrimer-Protease Adhäsion durch Bindung an ein Lectin hemmt.

7. Zusammensetzung, die eine Glykodendrimer-Protease nach einem der Ansprüche 1 bis 6 umfasst.

8. Zusammensetzung nach Anspruch 7, bei der es sich um eine Mundpflegezusammensetzung handelt.

9. Orale Pflegezusammensetzung nach Anspruch 8, worin die Mundpflegezusammensetzung eine Mundspülung oder Zahncreme ist.

10. Glykodendrimer-Protease nach einem der Ansprüche 1 bis 6 zur Verwendung in einem Verfahren zur Behandlung einer Krankheit bei einem Patienten, bei der angestrebt wird, die Adhäsion eines oder mehrerer Mikroorganismen an einer Oberfläche einer Körperöffnung zu hemmen.

11. Verwendung einer glykodendrimeren Protease nach einem der Ansprüche 1 bis 6 bei der Herstellung eines Medikaments zur Hemmung der Adhäsion von Mikroorganismen an einer Oberfläche.

12. Verwendung nach Anspruch 11, worin die Oberfläche aus der aus Zellen, Geweben, extrazellulärer Matrix, Zähnen und Prothesen bestehenden Gruppe ausgewählt ist.

13. Verwendung nach Anspruch 12, worin es sich bei der Oberfläche um orale Säugetierzellen oder -gewebe oder eine Zahnprothese handelt.

14. Verwendung nach Anspruch 13, worin die oralen Säugetiergewebe oder -zellen oder die Zahnprothese jene eines Menschen sind.

15. Verwendung nach Anspruch 11, worin das Medikament der topischen Verabreichung dient.

## Revendications

1. Protéase glycodendrimère, la protéase glycodendrimère ayant une structure ramifiée ou ramifiée multipliée qui présente un fragment de carbohydrate à chaque extrémité de ramification, où la protéase glycodendrimère inhibe l'adhérence d'un ou de plusieurs microorganismes à une surface,
où ladite protéase glycodendrimère a la structure 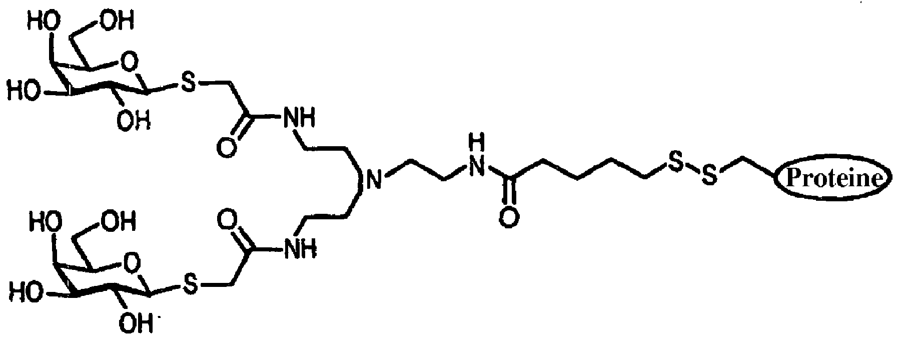 et où la protéase est la subtilisine *Bacillus lentus* S156C.

2. Protéase glycodendrimère selon la revendication 1, où la surface est sélectionnée dans le groupe consistant en cellules, tissus, matrice extracellulaire, dents et prothèses.

3. Protéase glycodendrimère selon la revendication 1, où le microorganisme comprend un procaryote, un eucaryote, un virus ou une combinaison de ceux-ci.

4. Protéase glycodendrimère selon la revendication 3, où le procaryote est une bactérie Gram-positive, Gram-négative ou Gram-variable.

5. Protéase glycodendrimère selon la revendication 4, où le procaryote est un *Actinomyces* ou *E*. *coli.*

6. Protéase glycodendrimère selon la revendication 1, où la protéase glycodendrimère inhibe l'adhérence par la liaison à une lectine.

7. Composition comprenant une protéase glycodendrimère selon l'une quelconque des revendications 1 à 6.

8. Composition selon la revendication 7, qui est une composition d'hygiène buccale.

9. Composition d'hygiène buccale selon la revendication 8, où ladite composition d'hygiène buccale est un rince-bouche ou un dentifrice.

10. Protéase glycodendrimère selon l'une quelconque des revendications 1 à 6, pour utilisation dans une méthode de traitement d'une maladie chez un patient où on cherche à inhiber l'adhérence d'un ou de plusieurs microorganismes à une surface d'une cavité corporelle.

11. Utilisation d'une protéase glycodendrimère selon l'une quelconque des revendications 1 à 6, dans la préparation d'un médicament pour inhiber l'adhérence de microorganismes à une surface.

12. Utilisation selon la revendication 11, où la surface est sélectionnée dans le groupe consistant en cellules, tissus, matrice extracellulaire, dents et prothèses.

13. Utilisation selon la revendication 12, où la surface sont des cellules ou tissus buccaux mammaliens, ou une prothèse dentaire.

14. Utilisation selon la revendication 13, où les tissus ou cellules buccaux mammaliens ou prothèse dentaire sont ceux d'un être humain.

15. Utilisation selon la revendication 11, où ledit médicament est prévu pour l'administration topique.
